# EUROPÄISCHE PATENTSCHRIFT

(11) **EP 0 625 515 B1**
(45) Veröffentlichungstag und Bekanntmachung des Hinweises auf die Patenterteilung: **11.08.1999**
(21) Anmeldenummer: 94106969.2
(22) Anmeldetag: 04.05.1994
(51) Int. Cl.: C07D 249/12, C07D 413/12, A01N 43/653, A01N 43/82

(54) **Substituierte Triazolinone sowie ihre Verwendung als Herbizide**
Substituted triazolinones and their use as herbicides
Triazolinones substituées et leur utilisation comme herbicides

(30) Priorität: 17.05.1993 DE 4316430
(43) Veröffentlichungstag der Anmeldung: 23.11.1994
(73) Patentinhaber: BAYER AG, 51368 Leverkusen (DE)
(72) Erfinder: Kluth, Joachim, Dr., D-40764 Langenfeld (DE); Müller, Klaus-Helmut, Dr., D-40593 Düsseldorf (DE); Haas, Wilhelm, Dr., D-50259 Pulheim (DE); Linker, Karl-Heinz, D-51377 Leverkusen (DE); Findeisen, Kurt, Prof. Dr., D-51375 Leverkusen (DE); König, Klaus, Dr., D-51519 Odenthal (DE); Santel, Hans-Joachim, Dr., D-51371 Leverkusen (DE); Dollinger, Markus, Dr., D-51381 Leverkusen (DE)

(56) Entgegenhaltungen:
- EP-A- 0 391 187
- EP-A- 0 422 469
- EP-A- 0 477 646
- PATENT ABSTRACTS OF JAPAN vol. 2, no. 24 (C-77)16. Februar 1978 & JP-A-52 125 168 (NIPPON SODA K.K.)

## Beschreibung

Die Erfindung betrifft neue substituierte Triazolinone, mehrere Verfahren zu ihrer Herstellung, mehrere neue Zwischenprodukte sowie ihre Verwendung als Herbizide.

Es ist bekannt, daß bestimmte substituierte Triazolinone wie beispielsweise die Verbindung 3 -Methylthio-4-amino-1-[N-(1,1-dimethylethyl)-aminocarbonyl]-1,2,4-triazolin-5-on herbizide Eigenschaften besitzen (vergl. z.B. EP 391 187). Siehe auch EP 477 646 und EP 422 469.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazolinone der allgemeinen Formel (I), 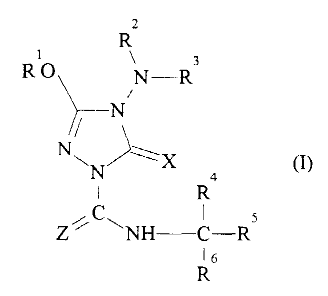 in welcher
- R¹: für Methyl, Ethyl, n- oder i-Propyl, n-, i-, s- oder t-Butyl, für Halogenalkyl mit 1 oder 2 Kohlenstoffatomen und 1 bis 5 gleichen oder verschiedenen Halogenatomen aus der Gruppe Fluor, Chlor und Brom , für Methoxymethyl, Methoxyethyl, für Cyclopropyl oder Cyclohexyl steht,
- R² und R³: entweder unabhängig voneinander jeweils für Wasserstoff, Methyl, Ethyl, n- oder i-Propyl, Methoxycarbonyl oder Ethoxycarbonyl stehen oder gemeinsam mit dem Stickstoffatom, an welches sie gebunden sind, für einen geradkettigen oder verzweigten Alkylideniminorest mit 1 bis 6 Kohlenstoffatomen oder für einen gegebenenfalls einfach bis dreifach, gleich oder verschieden substituierten Benzylideniminorest stehen, wobei als Substituenten im Arylteil infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
- R⁴: entweder für Wasserstoff, Cyano, Methyl, Ethyl, n- oder i-Propyl, n-, i-, s- oder t-Butyl, Allyl, Propargyl oder für geradkettiges oder verzweigtes Halogenalkyl mit 1 bis 6 Kohlenstoffatomen und 1 bis 13 gleichen oder verschiedenen Halogenatomen aus der Gruppe Fluor, Chlor und Brom steht und
- R⁵: für Wasserstoff, Methyl, Ethyl, n- oder i-Propyl, n-, i-, s- oder t-Butyl oder für geradkettiges oder verzweigtes Halogenalkyl mit 1 bis 6 Kohlenstoffatomen und 1 bis 13 gleichen oder verschiedenen Halogenatomen aus der Gruppe Fluor, Chlor und Brom steht oder
- R⁴ und R⁵: gemeinsam mit dem Kohlenstoffatom, an welches sie gebunden sind, für Cycloalkyl mit 3 bis 6 Kohlenstoffatomen stehen,
- R⁶: für Wasserstoff oder für gegebenenfalls einfach bis dreifach, gleich oder verschieden substituiertes, geradkettiges oder verzweigtes Alkyl mit 1 bis 7 Kohlenstoffatomen steht, wobei als Substituenten infrage kommen:
Halogen aus der Gruppe Fluor, Chlor und Brom, Cyano, Cycloalkyl mit 3 bis 6 Kohlenstoffatomen oder jeweils gegebenenfalls einfach bis dreifach, gleich oder verschieden substituiertes Phenyl, Phenoxy, Phenylthio, Phenylamino, jeweils einfach bis dreifach gleich oder verschieden substituiertes Heteroaryl, Heteroaryloxy, Heteroarylthio oder Heteroarylamino mit jeweils 1 bis 5 Kohlenstoffatomen und 1 bis 3 gleichen oder verschiedenen Heteroatomen aus der Gruppe Stickstoff, Sauerstoff und Schwefel im Heteroarylteil und wobei als Phenyl- bzw. Heteroarylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
- R⁶: außerdem für jeweils gegebenenfalls einfach bis dreifach, gleich oder verschieden substituiertes Phenyl oder Heteroaryl mit 1 bis 5 Kohlenstoffatomen und 1 bis 3 gleichen oder verschiedenen Heteroatomen aus der Gruppe Stickstoff, Sauerstoff und Schwefel steht, wobei als Phenyl- bzw. Heteroarylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
- X: für Sauerstoff und Schwefel steht und
- Z: für Sauerstoff und Schwefel steht.
gefunden.

Die Verbindungen der Formel (I) können gegebenenfalls in Abhängigkeit von der Art der Substituenten als optische Isomere oder Isomerengemische unterschiedlicher Zusammensetzung vorliegen. Sowohl die reinen Isomeren, als auch die Isomerengemische werden erfindungsgemäß beansprucht.

Weiterhin wurde gefunden, daß man die obengenannten Triazolinone erhält, wenn man
a) aktivierte (Thio)Carbonyltriazolinone der Formel (II), 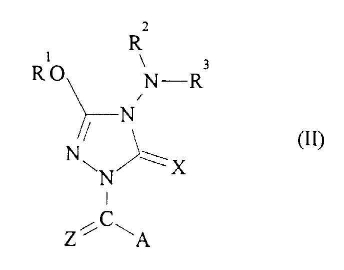 in welcher
   - R¹, R², R³, X und Z: die oben angegebenen Bedeutungen haben und
   - A: für eine Aktivierungsgruppe steht,
   mit Aminen der Formel (III), 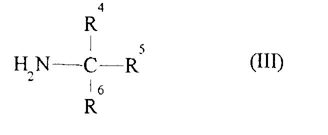 in welcher
   - R⁴, R⁵ und R⁶: die oben angegebenen Bedeutungen haben,
   gegebenenfalls in Gegenwart eines Verdünnungsmittels und gegebebenfalls in Gegenwart eines Reaktionshilfsmittels umsetzt, oder wenn man
b) in 1-Stellung unsubstituierte Triazolinone der Formel (IV), 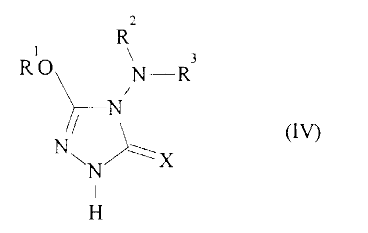 in welcher
   - R¹, R², R³ und X: die oben angegebenen Bedeutungen haben,
   mit Iso(thio)cyanaten der Formel (V), 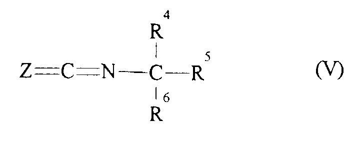 in welcher
   - R⁴, R⁵, R⁶ und Z: die oben angegebenen Bedeutungen haben,
   gegebenenfalls in Gegenwart eines Verdünnungsmittels und gegebenenfalls in Gegenwart eines Reaktionshilfsmittels umsetzt, oder wenn man
c) substituierte Triazolinone der Formel (Ia), 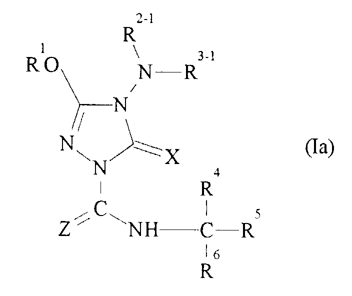 in welcher
   - R¹, R⁴, R⁵, R⁶, X und Z: die oben angegebenen Bedeutungen haben und
   - R²⁻¹ und R³⁻¹: gemeinsam mit dem Stickstoffatom, an welches sie gebunden sind, für einen gegebenenfalls substituierten Alkylideniminorest mit 1 bis 6 Kohlenstoffatomen stehen,
   mit Säure gegebenenfalls in Gegenwart eines Verdünnungsmittels und gegebenenfalls in Gegenwart eines Reaktionshilfsmittels umsetzt, oder wenn man
d) substituierte Triazolinone der Formel (Ib), 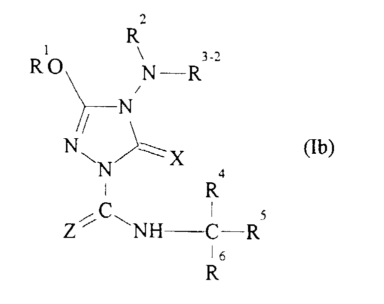 in welcher
   - R¹, R², R⁴, R⁵, R⁶, X und Z: die oben angegebenen Bedeutungen haben und
   - R³⁻²: für Methoxycarbonyl oder Ethoxycarbonyl steht,
   gegebenenfalls in Gegenwart eines Verdünnungsmittels und gegebenenfalls in Gegenwart eines Reaktionshilfsmittels verseift und anschließend thermisch decarboxyliert.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten substituierten Triazolinonen, wie beispielsweise die Verbindung 3-Methylthio-4-amino-1-[N-(t-butyl)-aminocarbonyl]-1,2,4-triazolin-5-on, welche chemisch und wirkungsmäßig naheliegende Verbindungen sind.

Im einzelnen seien außer den bei den Herstellungsbeispielen aufgeführten Verbindungen die folgenden substituierten Triazolinone der allgemeinen Formel (I) genannt:

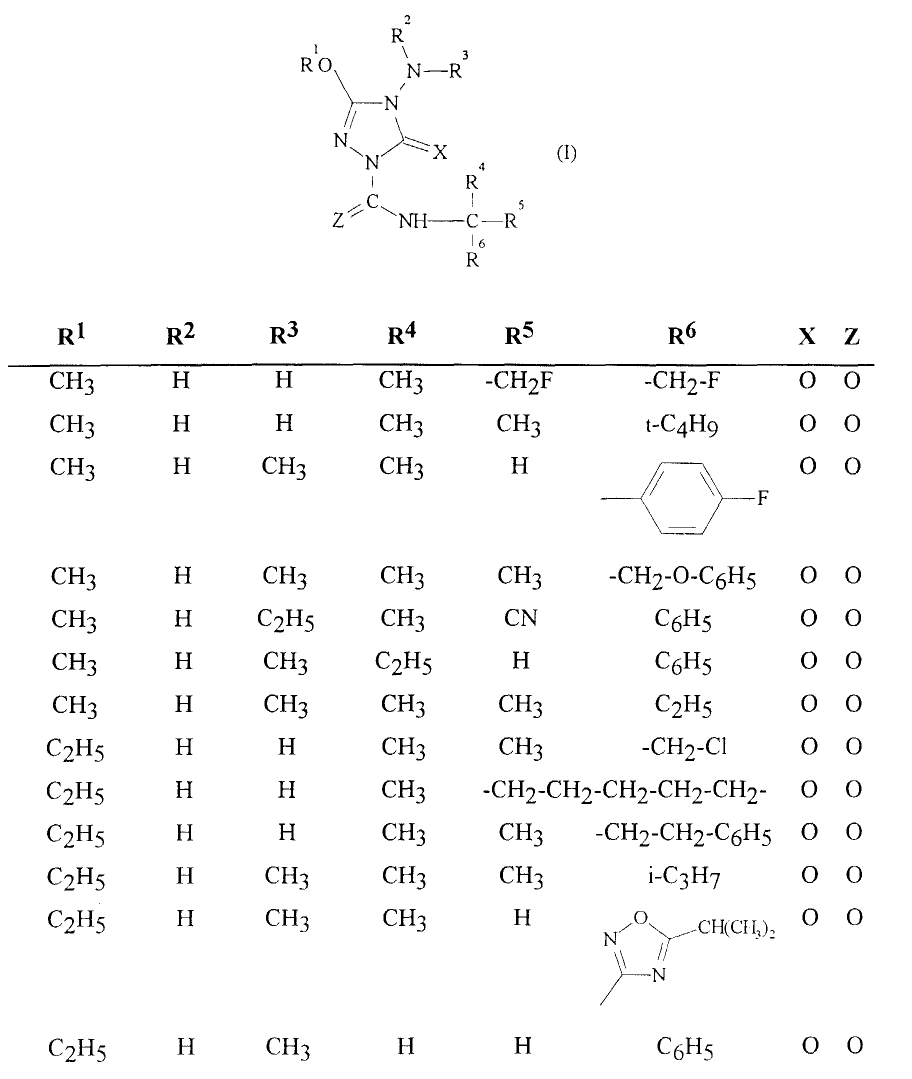

Verwendet man beispielsweise 1-Chlorcarbonyl-3-methoxy-4-isopropylidenimino1,2,4-triazolin-5-on und t-Butylamin als Ausgangsstoffe, so läßt sich der Reaktionsablauf des erfindungsgemäßen Verfahrens (a) durch das folgende Formelschema darstellen:

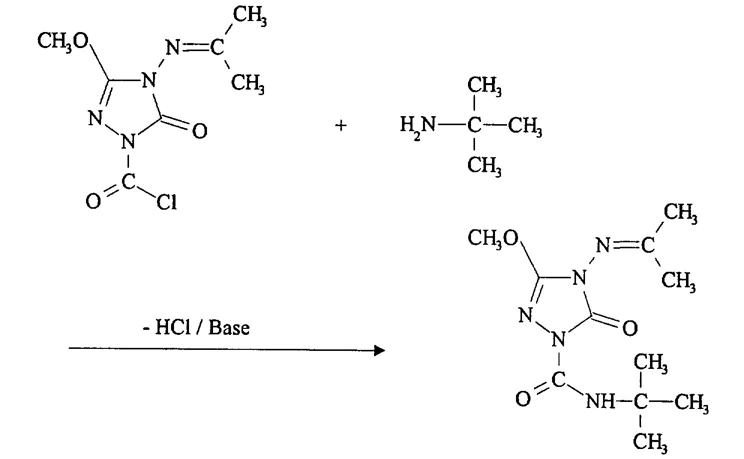

Verwendet man beispielsweise 3-Ethoxy-4-amino-1,2,4-triazolin-5-on und Chlor-t-butyl-isocyanat als Ausgangsstoffe, so läßt sich der Reaktionsablauf des erfindungsgemäßen Verfahrens (b) durch das folgende Formelschema darstellen:

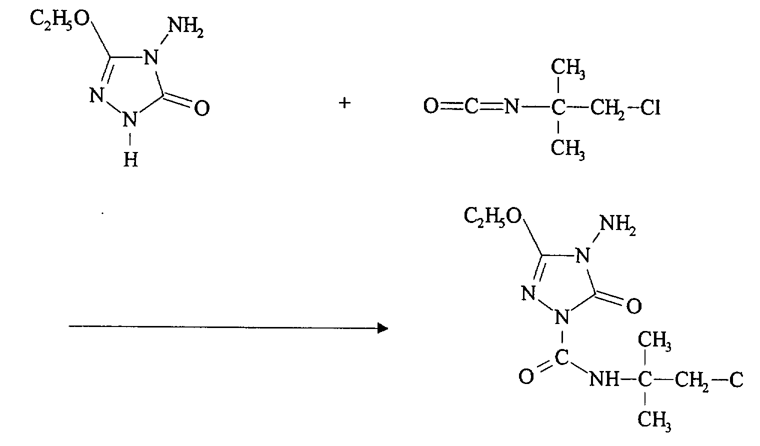

Verwendet man beispielsweise 1-[N-(1-Methyl-4-phenyl-but-2-yl)-amino]-carbonyl-3-n-propoxy-4-(4-methylpent-2-ylidenimino)-1,2,4-triazolin-5-on als Ausgangsver-bindung, so läßt sich der Reaktionsablauf des erfindungsgemäßen Verfahrens (c) durch das folgende Formelschema darstellen:

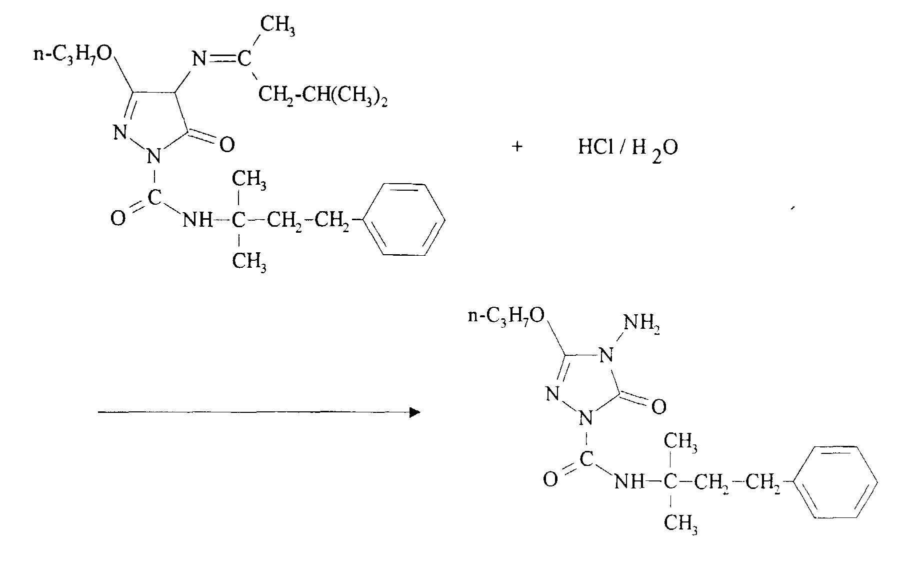

Verwendet man beispielsweise 1-(N-2-Methyl-3-cyclohexylprop-2-ylamino)-carbonyl-3-methoxy-4-(N-methyl-N-ethoxycarbonylamino)-1,2,4-triazolin-5-on als Ausgangsverbindung, so läßt sich der Reaktionsablauf des erfindungsgemäßen Verfahrens (d) durch das folgende Formelschema darstellen:

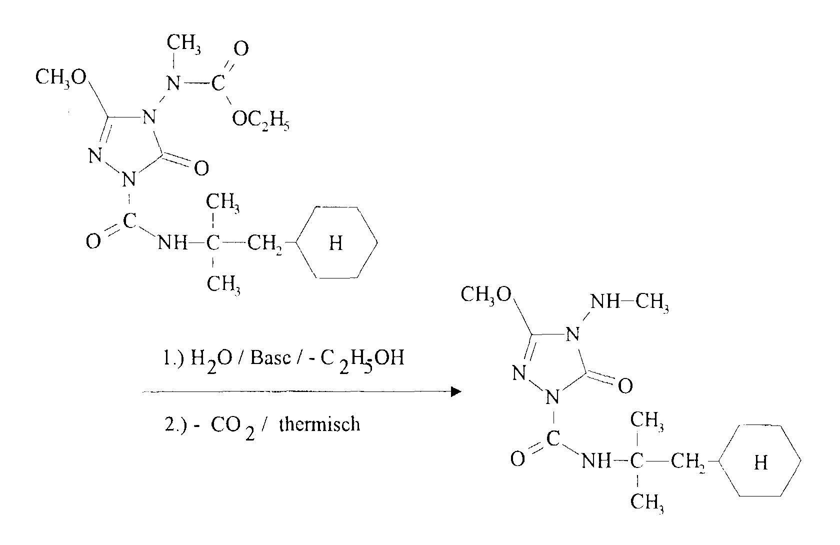

Die zur Durchführung des erfindungsgemäßen Verfahrens (a) als Ausgangsstoffe benötigten aktivierten (Thio)Carbonyltriazolinone sind durch die Formel (II) allgemein definiert. In dieser Formel (II) stehen R¹, R², R³, X und Z für diejenigen Reste, die bereits im Zusammenhang mit der Beschreibung der erfindungsgemäßen Verbindungen der Formel (I) für diese Substituenten genannt wurden. A steht für einen üblichen die benachbarte Carbonylgruppe aktivierenden Rest, insbesondere für Chlor, Phenoxy oder 1-Imidazolyl.

Die aktivierten (Thio)Carbonyltriazolinone der Formel (II) erhält man beispielsweise, wenn man in 1-Stellung unsubstituierte Triazolinone der Formel (IV), 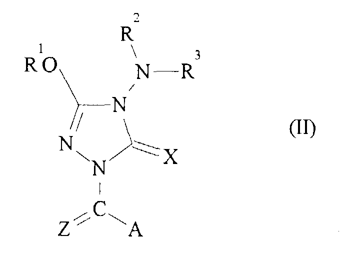 in welcher
- R¹, R², R³, X und Z: die oben angegebenen Bedeutungen haben und
- A: für eine Aktivierungsgruppe steht,
mit (Thio)Phosgen oder mit (Thio)Chlorameisensäurephenylester oder mit N,N'-Carbonyldiimidazol gegebenenfalls in Gegenwart eines Verdünnungsmittels wie beispielsweise Chloroform und gegebenenfalls in Gegenwart eines Reaktionshilfsmittels wie beispielsweise Triethylamin bei Temperaturen zwischen -20°C und +120°C umsetzt.

Die zur Durchführung des erfindungsgemäßen Verfahrens (a) weiterhin als Ausgangsstoffe benötigten Amine sind durch die Formel (III) allgemein definiert. In dieser Formel (III) stehen R⁴, R⁵ und R⁶ für diejenigen Reste, die bereits im Zusammenhang mit der Beschreibung der erfindungsgemäßen Verbindungen der Formel (I) für diese Substituenten genannt wurden.

Die Amine der Formel (III) sind allgemein bekannte Verbindungen der organischen Chemie oder erhältlich in Analogie zu allgemein bekannten Verfahren.

Die zur Durchführung des erfindungsgemäßen Verfahren (b) und zur Synthese der Vorprodukte der Formel (II) als Edukte erforderlichen in 1-Stellung unsubstituierten Triazolinone sind durch die Formel (IV) allgemein definiert. In dieser Formel (IV) stehen R¹, R², R³ und X für diejenigen Reste, die bereits im Zusammenhang mit der Beschreibung der erfindungsgemäßen Stoffe der Formel (I) für diese Substituenten genannt wurden.

Die in 1-Stellung unsubstituierten Triazolinone der Formel (IV) sind bekannt (vergl. z.B. EP 422 469; J. Chem. Soc. C, 1967, 2700-2704; Arm. Khim. Zh. 29, 545-547 [1976] bzw. CA 85:159998h).

Man erhält bekannte die in 1-Stellung unsubstituierte Triazolinone der Formel (IV) in Analogie zu bekannten Verfahren (vergl. z.B. EP 422 469; J. Chem. Soc. C, 1967, 2700-2704; Arm. Khim. Zh. 29, 545-547 [1976] bzw. CA 85:159998h) beispielsweise, wenn man (Thio)Carbodihydrazide der Formel (VI), 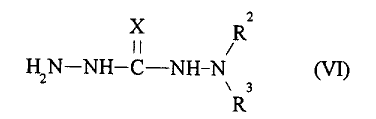 in welcher
- R², R³ und X: die oben angegebenen Bedeutungen haben,
mit Kohlensäureorthoestern der Formel (VII),

C(OR¹)₄ (VII)

in welcher
- R¹: die oben angegebene Bedeutung hat,
gegebenenfalls in Gegenwart eines Verdünnungsmittels wie beispielsweise Methanol oder Ethanol bei Temperaturen zwischen 0°C und 120°C umsetzt.

(Thio)Carbodihydrazide der Formel (VI) und Orthoester der Formel (VII) sind allgemein bekannte Verbindungen der organischen Chemie oder erhältlich mit Hilfe allgemein bekannter Verfahren.

Die zur Durchführung des erfindungsgemäßen Verfahrens (b) weiterhin als Edukte erforderlichen Iso(thio)cyanate sind durch die Formel (V) allgemein definiert. In dieser Formel (V) stehen R⁴, R⁵, R⁶ und Z für diejenigen Reste, die bereits im Zusammenhang mit der Beschreibung der erfindungsgemäßen Stoffe der Formel (I) für diese Substituenten genannt wurden.

Die Iso(thio)cyanate der Formel (V) sind allgemein bekannte Verbindungen der organischen Chemie oder erhältlich in Analogie zu allgemein bekannten Verfahren.

Die zur Durchführung des erfindungsgemäßen Verfahrens (c) als Edukte erforderlichen substituierten Triazolinone sind durch die Formel (Ia) allgemein definiert. In dieser Formel (Ia) stehen R¹, R⁴, R⁵, R⁶, X und Z für diejenigen Reste, die bereits im Zusammenhang mit der Beschreibung der erfindungsgemäßen Stoffe der Formel (I) für diese Substituenten genannt wurden. R²⁻¹ und R³⁻¹ stehen gemeinsam mit dem Stickstoffatom, an welches sie gebunden sind für einen geradkettigen oder verzweigten Alkylideniminorest mit 1 bis 6 Kohlenstoffiatomen oder für einen gegebenenfalls einfach oder dreifach, gleich oder verschieden substituierten Benzylideniminorest, wobei als Substituenten im Arylteil in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl.

Die substituierten Triazolinone der Formel (Ia) sind erfindungsgemäße Verbindungen und erhältlich mit Hilfe der erfindungsgemäßen Verfahren (a) oder (b).

Die zur Durchführung des erfindungsgemäßen Verfahren (d) als Edukte erforderlichen substituierten Triazolinone sind durch die Formel (Ib) allgemein definiert. In dieser Formel (Ib) stehen R¹, R², R⁴, R⁵, R⁶, X und Z für diejenigen Reste, die bereits im Zusammenhang mit der Beschreibung der erfindungsgemäßen Stoffe der Formel (I) für diese Substituenten genannt wurden. R³⁻² steht für Methoxycarbonyl oder Ethoxycarbonyl.

Die substituierten Triazolinone der Formel (Ib) sind erfindungsgemäße Verbindungen und erhältlich mit Hilfe der erfindungsgemäßen Verfahren (a) oder (b).

Als Verdünnungsmittel zur Durchführung des erfindungsgemäßen Verfahrens (a) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Methyl-t-butylether, Methyl-t-pentylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Basen wie Pyridin.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (III) in entsprechendem Überschuß gleichzeitig als Reaktionshilfsmittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +150°C, vorzugsweise bei Temperaturen zwischen +10°C und +80°C.

Zur Durchführung des erfindungsgemäßen Verfahrens (a) setzt man pro Mol aktiviertem (Thio)Carbonyltriazolinon der Formel (II) im allgemeinen 1,0 bis 5,0 Mol, vorzugsweise 1,0 bis 2 Mol Amin der Formel (III) und gegebenenfalls 1,0 bis 5,0 Mol, vorzugsweise 1,0 bis 2 Mol Base als Reaktionshilfsmittel ein. Die Reaktionsdurchführung, Aufarbeitung und Isolierung der Reaktionsprodukte erfolgt nach bekannten Verfahren (vergl. hierzu beispielsweise EP 283 876 oder die Herstellungsbeispiele).

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Das erfindungsgemäße Verfahren (b) kann gegebenenfalls in Gegenwart eines geeigneten basischen Reaktionshilfsmittels durchgeführt werden. Als solche kommen alle üblichen anorganischen oder organischen Basen in Frage. Vorzugsweise verwendet man tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Der Zusatz derartig basischer Reaktionshilfsmittel ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und +150°C, vorzugsweise bei Temperaturen zwischen +40°C und +120°C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol in 1-Stellung unsubstituiertem Triazolinon der Formel (IV) im allgemeinen 1,0 bis 5,0 Mol, vorzugsweise 1,0 bis 2,5 Mol Iso(thio)cyanat der Formel (V) und gegebenenfalls 0,01 bis 5,0 Mol, vorzugsweise 0,1 bis 2,5 Mol Base als Reaktionshilfsmittel ein.

Die Reaktionsdurchführung, Aufarbeitung und Isolierung der Reaktionsprodukte erfolgt nach bekannten Verfahren (vergl. hierzu beispielsweise EP 283 876 oder die Herstellungsbeispiele).

Als Säuren kommen zur Durchführung des erfindungsgemäßen Verfahrens (c) alle üblicherweise für Hydrazonspaltungen verwendbaren anorganischen und organischen Säuren infrage. Vorzugsweise verwendet man anorganische Mineralsäuren wie Chlorwasserstoffsäure, Schwefelsäure und Phosphorsäure oder saure Ionenaustauscher.

Als Verdünnungsmittel kommen zur Durchführung des erfindungsgemäßen Verfahrens (c) alle üblichen organischen oder anorganischen Lösungsmittel infrage. Vorzugsweise verwendet man polare mit Wasser mischbare organische Lösungsmittel, insbesondere Alkohole, wie Methanol, Ethanol, Propanol oder Butanol, deren Gemische mit Wasser oder reines Wasser als Lösungsmittel.

Die Reaktionstemperaturen können bei der Durchführung des erfindungsgemäßen Verfahrens (c) in einem größeren Bereich variiert werden. Im allgemeinen arbeitet man bei Temperaturen zwischen +20°C und +150°C, vorzugsweise bei Temperaturen zwischen +50°C und +120°C.

Das erfindungsgemäße Verfahren (c) wird üblicherweise unter Normaldruck oder unter vermindertem Druck durchgeführt. Arbeitet man unter vermindertem Druck, so kommen Druckbereiche zwischen 20 und 400 mbar, vorzugsweise zwischen 100 und 200 mbar infrage.

Zur Durchführung des erfindungsgemäßen Verfahrens (c) setzt man pro Mol substituiertem Triazolinon der Formel (Ia) im allgemeinen 1,0 bis 5,0 Mol, vorzugsweise 1,0 bis 2,5 Mol Säure ein. Dabei löst man das substituierte Triazolinon der Formel (Ia) in einer geeigneten Menge an Verdünnungsmittel, setzt dann die erforderliche Menge Säure zu und engt die Mischung unter vermindertem Druck über mehrere Stunden langsam ein. In einer besonderen Durchführungsform ist es auch möglich, das erfindungsgemäße Verfahren (c) und die Herstellung der dafür erforderlichen Vorprodukte der Formel (Ia) in einem Reaktionsschritt in einem sogenannten "Eintopfverfahren" durchzuführen. Dabei geht man so vor, daß man als Ausgangsverbindungen entweder die aktivierten (Thio)Carbonyltriazolinone der Formel (II) wählt und diese nacheinander im "Eintopfverfahren" mit Aminen der Formel (III) gemäß dem erfindungsgemäßen Verfahren (a) und anschließend mit Säure gemäß dem erfindungsgemäßen Verfahren (c) umsetzt oder alternativ, daß man als Ausgangsverbindungen die in 1-Stellung unsubstituierten Triazolinone der Formel (IV) wählt und diese nacheinander im "Eintopfverfahren" mit Iso(thio)cyanaten der Formel (V) gemäß dem erfindungsgemäßen Verfahren (b) und anschließend mit Säure gemäß dem erfindungsgemäßen Verfahren (c) umsetzt.

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Methyl-t-butylether, Methyl-t-pentylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Das erfindungsgemäße Verfahren (d) kann gegebenenfalls in Gegenwart eines geeigneten Reaktionshilfsmittels durchgeführt. Als solche kommen übliche anorganische oder organische Säuren oder Basen infrage. Hierzu gehören beispielsweise Mineralsäuren, wie Salzsäure oder Schwefelsäure, Carbonsäuren, wie Essigsäure, Sulfonsäuren, wie p-Toluolsulfonsäure, Erdalkali- oder Alkalimetallhydroxide, -alkoholate, -acetate, -carbonate oder -hydrogencarbonate, wie beispielsweise Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU) oder saure bzw. basische Ionenaustauscher.

Die Reaktionstemperaturen können bei der Durchführung des erfindungsgemäßen Verfahrens (d) in einem größeren Bereich variiert werden. Im allgemeinen arbeitet man bei Temperaturen zwischen 50°C und 200°C, vorzugsweise bei Temperaturen zwischen 80°C und 180°C.

Zur Durchführung des erfindungsgemäßen Verfahrens (d) setzt man pro Mol substituiertem Triazolinon der Formel (Ib) gegebenenfalls im allgemeinen 0,01 bis 2,0 Mol, vorzugsweise 0,1 bis 1,0 Mol saures oder basisches Reaktionshilfsmittel ein. Die Reaktionsdurchführung, Aufarbeitung und Isolierung der Reaktionsprodukte erfolgt in Analogie zu bekannten Verfahren (vergl. hierzu beispielsweise Houben-Weyl "Methoden der organischen Chemie", Band XI,1; S.863, 867, 948; Thieme Verlag Stuttgart).

Die Reaktionsdurchführung, Aufarbeitung und Isolierung der Reaktionsprodukte erfolgt jeweils in Analogie zu bekannten Verfahren (vergl. hierzu beispielsweise EP 283 876; EP 370 293 oder die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe des Brechungsindex oder der Protonen-Kernresonanzspektroskopie (¹H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern in monokotylen Kulturen wie beispielsweise Mais oder Weizen einsetzen.

Die Wirkstoffe können in die üblichen Formulierungen überführt werden, wie Lösungen, Emulsionen, Suspensionen, Pulver, Schäume, Pasten, Granulate, Aerosole, Wirkstoff-imprägnierte Natur- und synthetische Stoffe, sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere venvendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

### Herstellungsbeispiele:

### Beispiel 1

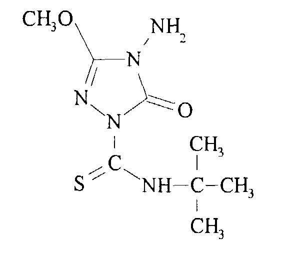

### (Verfahren a)

Zu 2,7 g (0,01 Mol) 4-Amino-3-methoxy-1-(phenoxy-thiocarbonyl)-1,2,4-triazolin-5-on in 100 ml Tetrahydrofuran gibt man bei Raumtemperatur unter Rühren 2,2 g (0,03 Mol) t-Butylamin und rührt nach beendeter Zugabe 16 Stunden bei Rückflußtemperatur. Zur Aufarbeitung wird auf Raumtemperatur abgekühlt, ausgefallener Niederschlag abfiltriert, das Filtrat eingeengt und der Rückstand durch Verreiben mit Diethylether zur Kristallisation gebracht.

Man erhält 1,8 g (74 % der Theorie) 4-Amino-3-methoxy-1-[N-(2-Methylprop-2-yl)-amino]-carbonyl-1,2,4-triazolin-5-on vom Schmelzpunkt 185°C.

### Beispiel 2

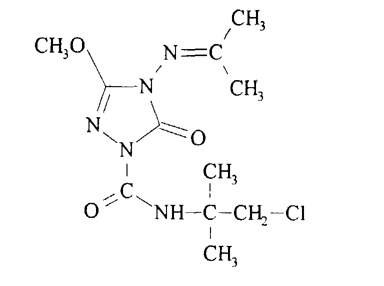

### (Verfahren b)

Zu 2,55 g (0,015 Mol) 4-Isopropylidenimino-3-methoxy-1,2,4-triazolin-5-on in 50 ml Acetonitril gibt man bei Raumtemperatur tropfenweise unter Rühren 4,0 g (0,03 Mol) Chlor-t-butylisocyanat und rührt nach beendeter Zugabe 24 Stunden bei Raumtemperatur. Zur Aufarbeitung wird ausgefallener Niederschlag abgesaugt, mit Diethylether gewaschen, getrocknet und über Kieselgel (Laufmittel: Cyclohexan/Essigester 1:3) chromatographiert.

Man erhält 4 g (89 % der Theorie) 4-Isopropylidenimino-3-methoxy-1-[N-(3-chlor-2-methylprop-2-yl)-amino]-carbonyl-1,2,4-triazolin-5-on als Öl.

¹H-NMR (CDCl₃/Tetramethylsilan): δ = 7,85 ppm (NH).

### Beispiel 3

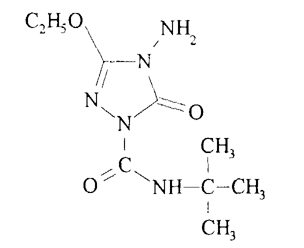

### (Verfahren b)

Zu 4,3 g (0,03 Mol) 4-Amino-3-ethoxy-1,2,4-triazolin-5-on in 100 ml Acetonitril gibt man bei Raumtemperatur tropfenweise unter Rühren 3,0 g (0,03 Mol) t-Butylisocyanat und 10 Tropfen Diazabicycloundecen (DBU) und rührt nach beendeter Zugabe 16 Stunden bei Raumtemperatur. Zur Aufarbeitung wird die Reaktionsmischung im Vakuum eingeengt, der Rückstand in Dichlormethan aufgenommen mit Wasser neutral gewaschen, getrocknet, im Vakuum eingeengt und der Rückstand durch Verreiben mit Diethylether zur Kristallisation gebracht.

Man erhält 2,7 g (37 % der Theorie) 4-Amino-3-ethoxy-1-[N-(2-methylprop-2-yl)-amino]-carbonyl-1,2,4-triazolin-5-on vom Schmelzpunkt 124°C.

### Beispiel 4

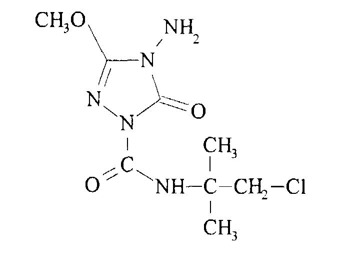

### (Verfahren (c)

Zu 3,5 g (0,0116 Mol) 4-Isopropylidenimino-3-methoxy-1-[N-(chlor-t-butyl)-amino]-carbonyl-1,2,4-triazolin-5-on in 30 ml Ethanol gibt man bei Raumtemperatur unter Rühren 20 ml Wasser und 5 Tropfen verdünnte Salzsäure, erwärmt anschließend für 60 Minuten auf 40°C, engt dann im Vakuum ein und reinigt den Rückstand durch Chromatographie an Kieselgel (Laufmittel: Essigester).

Man erhält 1,8 g (59 % der Theorie) 4-Amino-3-methoxy-1-[N-(3-chlor-2-methyl-prop-2-yl)-amino]-carbonyl-1,2,4-triazolin-5-on vom Schmelzpunkt 118-120°C.

In entsprechender Weise und gemäß den allgemeinen Angaben zur Herstellung erhält man die folgenden substituierten Triazolinone der allgemeinen Formel (I):

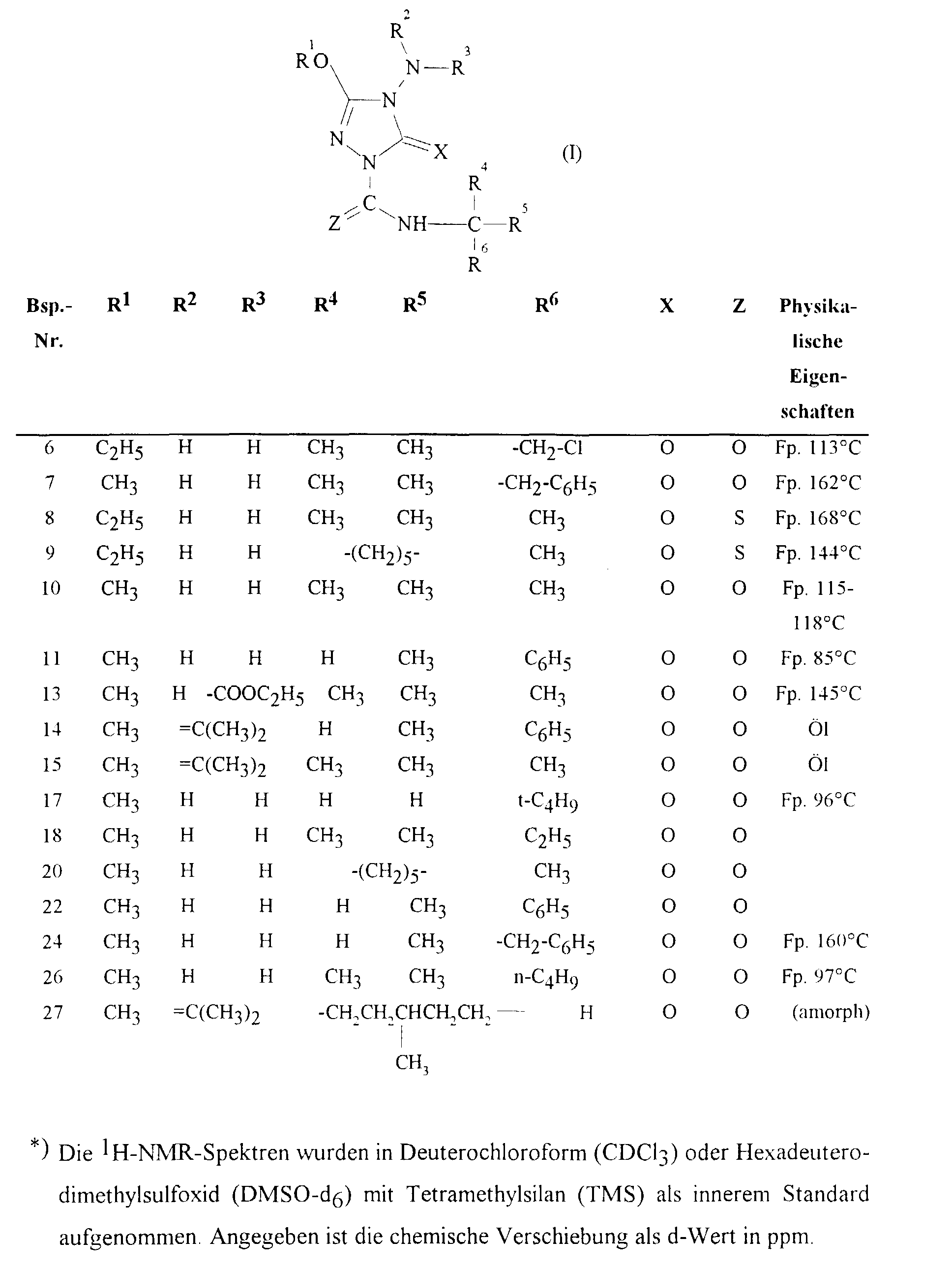

### Herstellung der Ausgangsverbindungen:

### Beispiel II-1

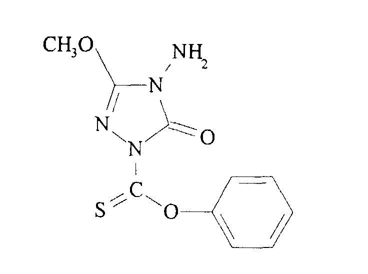

Zu 1,3 g (0,01 Mol) 4-Amino-3-methoxy-1,2,4-triazolin-5-on in einer Mischung aus 50 ml Dichlormethan, 50 ml Wasser, 0,5 g (0,0125 Mol) Natriumhydroxid und 100 mg Tetrabutylammoniumbromid gibt man bei Raumtemperatur tropfenweise unter Rühren 1,9 g (0,011 Mol) Thiochlorameisensäure-O-phenylester und rührt anschließend weitere 15 Stunden bei Raumtemperatur. Zur Aufarbeitung wird die organische Phase abgetrennt, mit verdünnter Salzsäure gewaschen, getrocknet, im Vakuum eingeengt und der Rückstand durch Verreiben mit Ether/Isopropanol (10:1) zur Kristallisation gebracht.

Man erhält 0,5 g (19 % der Theorie) 4-Amino-3-methoxy-1-phenoxy-thiocarbonyl-1,2,4-triazolin-5-on vom Schmelzpunkt 158°C.

In entsprechender Weise und gemäß den allgemeinen Angaben zur Herstellung erhält man die folgenden aktivierten (Thio)Carbonyltriazolinone der Formel (II):

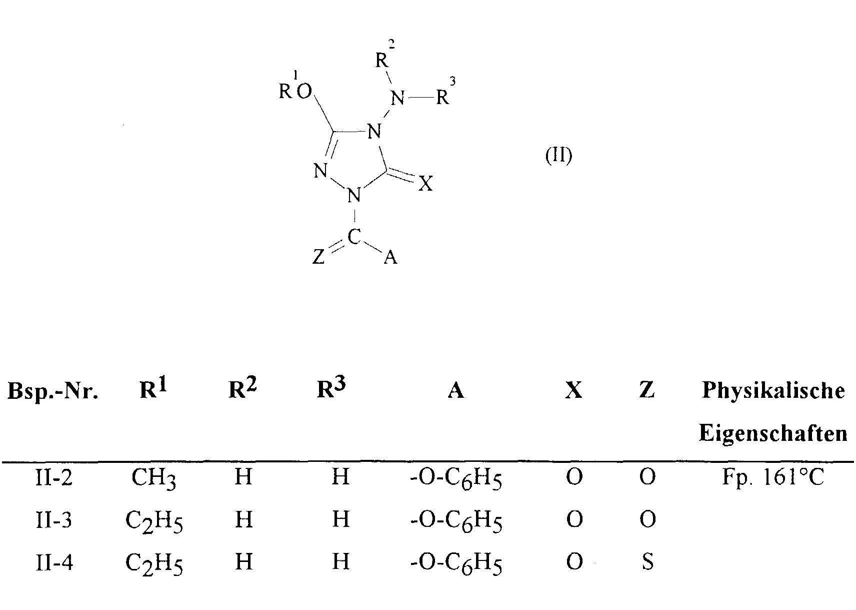

### Beispiel IV-1:

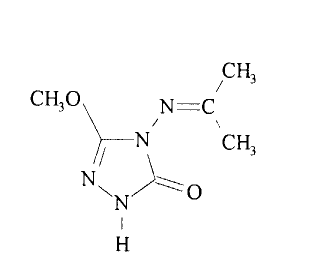

Zu 13 g (0,1 Mol) 4-Amino-3-methoxy-1,2,4-triazolin-5-on in 200 ml Aceton gibt man katalytische Mengen p-Toluolsulfonsäure, erwärmt anschließend für 12 Stunden auf Rückflußtemperatur, wobei kontinuierlich Aceton/Wasser-Gemisch abdestilliert wird. Zur Aufarbeitung wird die resultierende Lösung heiß filtriert, das Filtrat anschließend im Vakuum eingeengt und der Rückstand durch Chromatographie an Kieselgel (Laufmittel: Aceton) gereinigt.

Man erhält 14 g (82 % der Theorie) 4-Isopropylidenimino-3-methoxy-1H-1,2,4-triazolin-5-on als Öl.
¹H-NMR (CDCl₃/Tetramethylsilan): δ = 9,7 ppm (NH).

### Beispiel IV-2:

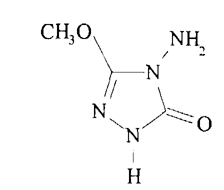

Zu 54 g (0,6 Mol) Carbodihydrazid in 300 ml Methanol gibt man 81,6 g (0,6 Mol) o-Kohlensäuretetramethylester, rührt anschließend 24 Stunden bei Raumtemperatur und weitere 24 Stunden bei Rückflußtemperatur. Zur Aufarbeitung wird die Reaktionsmischung abgekühlt, im Vakuum auf ein Drittel des Volumens eingeengt, der ausgefallene Feststoff abfiltriert, mit Diethylether gewaschen und getrocknet.

Man erhält 38 g (49 % der Theorie) 4-Amino-3-methoxy-lH-1,2,4-triazolin-5-on.

In entsprechender Weise und gemäß den allgemeinen Angaben zur Herstellung erhält man die folgenden in 1-Stellung unsubstituierten Triazolinone der allgemeinen Formel (IV):

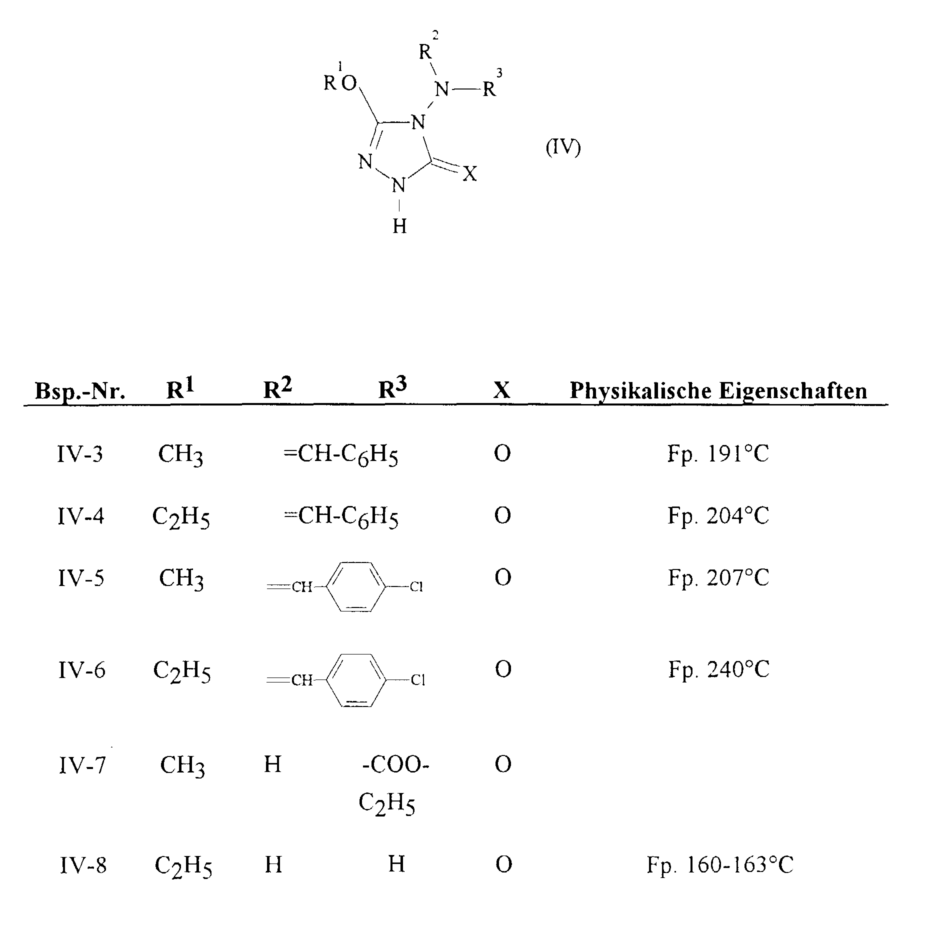

### Anwendungsbeispiele:

In den folgenden Anwendungsbeispielen wurde die nachstehend aufgeführte Verbindung als Vergleichssubstanz eingesetzt: 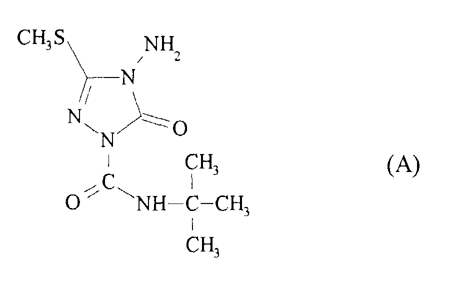 3 -Methylthio-4-amino-1-[N-(t-butyl)-aminocarbonyl]-1,2,4-triazolin-5-on (bekannt aus EP 391 187)

### Beispiel A

### Pre-emergence-Test

- Lösungsmittel:: 5 Gewichtsteile Aceton
- Emulgator:: 1 Gewichtsteil Alkylarylpolyglykolether
Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

Eine deutliche Überlegenheit in der Wirksamkeit bei vergleichbarer Nutzpflanzenselektivität gegenüber dem Stand der Technik zeigt in diesem Test z.B. die Verbindung gemäß Herstellungsbeispiel 10 in Maiskulturen gegenüber Unkräutern wie Abutidon (95 %), Cassia (90 %), Chenopodium (90 %), Galinsoga (95 %) Sinapis (100 %) und Viola (90 % bei einer Aufwandmenge von 125 g/ha.

### Beispiel B:

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Die Konzentration der Spritzbrühe wird so gewählt, daß in 2000 1 Wasser pro Hektar die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100% =: totale Vernichtung

Eine deutliche Überlegenheit in der Wirksamkeit bei vergleichbarer Nutzpflanzenselektivität gegenüber dem Stand der Technik zeigt in diesem Test z.B. die Verbindung gemäß Herstellungsbeispiel 10 in Weizenkulturen gegenüber Unkräutern wie Amaranthus (95 %), Solanum (100 %), Veronica (100 %), Viola (7o %) und Xanthium (100 %) bei einer Aufwandmenge von 125 g/ha..

## Patentansprüche

1. Substituierte Triazolinone der allgemeinen Formel (I) 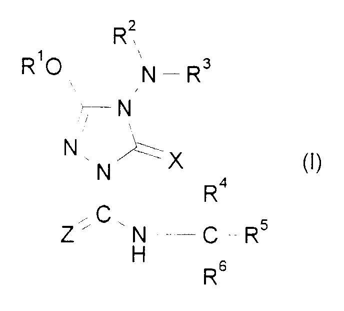 in welcher
R¹ für Methyl, Ethyl, n- oder i-Propyl, n-, i-, s- oder t-Butyl, für Halogenalkyl mit 1 oder 2 Kohlenstoffatomen und 1 bis 5 gleichen oder verschiedenen Halogenatomen aus der Gruppe Fluor, Chlor und Brom, für Methoxymethyl, Methoxyethyl, für Cyclopropyl oder Cyclohexyl steht,
R² und R³ entweder unabhängig voneinander jeweils für Wasserstoff, Methyl, Ethyl, n- oder i-Propyl, Methoxycarbonyl oder Ethoxycarbonyl stehen oder gemeinsam mit dem Stickstoffatom, an welches sie gebunden sind, für einen geradkettigen oder verzweigten Alkylideniminorest mit 1 bis 6 Kohlenstoffatomen oder für einen gegebenenfalls einfach bis dreifach, gleich oder verschieden substituierten Benzylideniminorest stehen, wobei als Substituenten im Arylteil infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
R⁴ entweder für Wasserstoff, Cyano, Methyl, Ethyl, n- oder i-Propyl, n-, i-, s- oder t-Butyl, Allyl, Propargyl oder für geradkettiges oder verzweigtes Halogenalkyl mit 1 bis 6 Kohlenstoffatomen und 1 bis 13 gleichen oder verschiedenen Halogenatomen aus der Gruppe Fluor, Chlor und Brom steht und
R⁵ für Wasserstoff, Methyl, Ethyl, n- oder i-Propyl, n-, i-, s- oder t-Butyl oder für geradkettiges oder verzweigtes Halogenalkyl mit 1 bis 6 Kohlenstoffatomen und 1 bis 13 gleichen oder verschiedenen Halogenatomen aus der Gruppe Fluor, Chlor und Brom steht oder
R⁴ und R⁵ gemeinsam mit dem Kohlenstoffatom, an welches sie gebunden sind, für Cycloalkyl mit 3 bis 6 Kohlenstoffatomen stehen,
R⁶ für Wasserstoff oder für gegebenenfalls einfach bis dreifach, gleich oder verschieden substituiertes, geradkettiges oder verzweigtes Alkyl mit 1 bis 7 Kohlenstoffatomen steht, wobei als Substituenten infrage kommen:
Halogen aus der Gruppe Fluor, Chlor und Brom, Cyano, Cycloalkyl mit 3 bis 6 Kohlenstoffatomen oder jeweils gegebenenfalls einfach bis dreifach, gleich oder verschieden substituiertes Phenyl, Phenoxy. Phenylthio, Phenylamino, jeweils einfach bis dreifach gleich oder verschieden substituiertes Heteroaryl, Heteroaryloxy, Heteroarylthio oder Heteroarylamino mit jeweils 1 bis 5 Kohlenstoffatomen und 1 bis 3 gleichen oder verschiedenen Heteroatomen aus der Gruppe Stickstoff, Sauerstoff und Schwefel im Heteroarylteil und wobei als Phenyl-bzw. Heteroarylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
R⁶ außerdem für jeweils gegebenenfalls einfach bis dreifach, gleich oder verschieden substituiertes Phenyl oder Heteroaryl mit 1 bis 5 Kohlenstoffatomen und 1 bis 3 gleichen oder verschiedenen Heteroatomen aus der Gruppe Stickstoff, Sauerstoff und Schwefel steht, wobei als Phenyl- bzw. Heteroarylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl,
X für Sauerstoff oder Schwefel steht und
Z für Sauerstoff oder Schwefel steht.

2. Verfahren zur Herstellung substituierter Triazolinone gemäß Anspruch 1, dadurch gekennzeichnet, daß man
a) aktivierte (Thio)Carbonyltriazolinone der Formel (II), 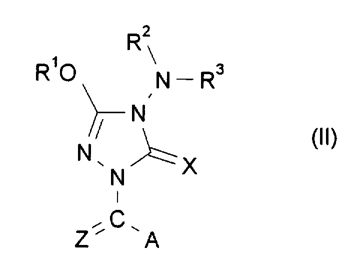 in welcher
R¹, R², R³, X und Z die in Anspruch 1 angegebenen Bedeutungen haben und
A für eine Aktivierungsgruppe steht,
mit Aminen der Formel (III), 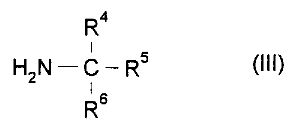 in welcher
R⁴, R⁵ und R⁶ die in Anspruch 1 angegebenen Bedeutungen haben,
gegebenenfalls in Gegenwart eines Verdünnungsmittels und gegebenenfalls in Gegenwart eines Reaktionshilfsmittels umsetzt, oder daß man
b) in 1-Stellung unsubstituierte Triazolinone der Formel (IV), 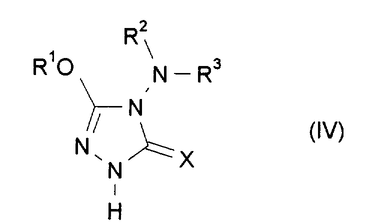 in welcher
R¹, R², R³ und X die in Anspruch 1 angegebenen Bedeutungen haben,
mit Iso(thio)cyanaten der Formel (V), 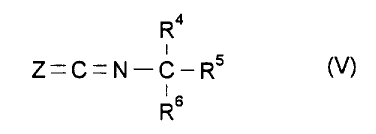 in welcher
R⁴, R⁵, R⁶ und Z die in Anspruch 1 angegebenen Bedeutungen haben,
gegebenenfalls in Gegenwart eines Verdünnungsmittels und gegebenenfalls in Gegenwart eines Reaktionshilfsmittels umsetzt, oder daß man
c) substituierte Triazolinone der Formel (Ia), 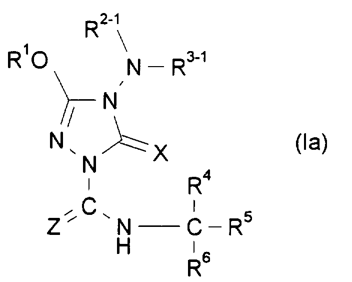 in welcher
R¹, R⁴, R⁵, R⁶, X und Z die in Anspruch 1 angegebenen Bedeutungen haben und
R²⁻¹ und R³⁻¹ gemeinsam mit dem Stickstoffatom, an welches sie gebunden sind, für einen gegebenenfalls substituierten Alkylideniminores mit 1 bis 6 Kohlenstoffatomen stehen,
mit Säure gegebenenfalls in Gegenwart eines Verdünnungsmittels und gegebenenfalls in Gegenwart eines Reaktionshilfsmittels umsetzt, oder daß man
d) substituierte Triazolinone der Formel (Ib), 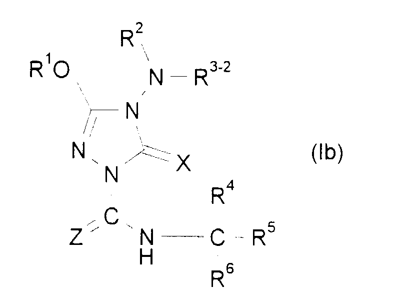 in welcher
R¹, R², R⁴, R⁵, R⁶, X und Z die in Anspruch 1 angegebenen Bedeutungen haben und
R³⁻² für Methoxycarbonyl oder Ethoxycarbonyl steht,
gegebenenfalls in Gegenwart eines Verdünnungsmittels und gegebenenfalls in Gegenwart eines Reaktionshilfsmittels verseift und anschließend thermisch decarboxyliert.

3. Herbizide Mittel, gekennzeichnet durch einen Gehalt an mindestens einem substituierten Triazolinon gemäß Anspruch 1.

4. Verfahren zur Bekämpfung von unerwünschten Pflanzen, dadurch gekennzeichnet, daß man substituierte Triazolinone gemäß Anspruch 1 auf Pflanzen und/oder ihren Lebensraum einwirken läßt.

5. Verwendung von substituierten Triazolinonen gemäß Anspruch 1 zur Bekämpfung von unerwünschten Pflanzen.

6. Verfahren zur Herstellung von herbiziden Mitteln, dadurch gekennzeichnet, daß man substituierte Triazolinone gemäß Anspruch 1 mit Streckmitteln und/oder oberflächenaktiven Substanzen vermischt.

7. Substituierte Triazolinone der Formel (Ia), 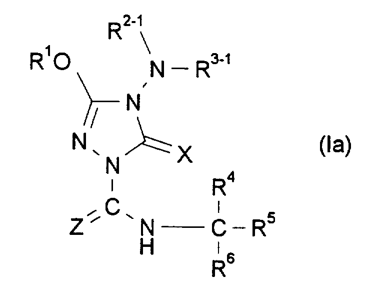 in welcher
R¹, R⁴, R⁵, R⁶, X und Z die in Anspruch 1 angegebenen Bedeutungen haben und
R²⁻¹ und R³⁻¹ gemeinsam mit dem Stickstoffatom, an welches sie gebunden sind, für einen geradkettigen oder verzweigten Alkylideniminorest mit 1 bis 6 Kohlenstoffatomen stehen.

8. Substituierte Triazolinone der Formel (Ib), 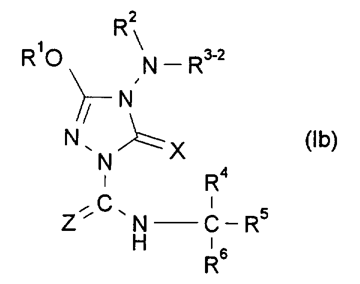 in welcher
R¹, R², R⁴, R⁵, R⁶, X und Z die in Anspruch 1 angegebenen Bedeutungen haben und
R³⁻² für Methoxycarbonyl oder Ethoxycarbonyl steht.

## Claims

1. Substituted triazolinones of the general formula (I) 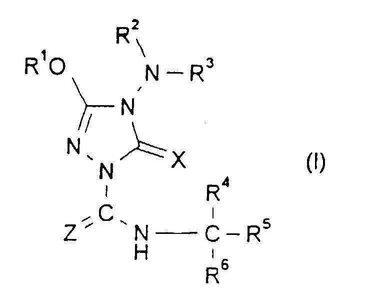 in which
R¹ represents methyl, ethyl, n- or i-propyl, n-, i-, s- or t-butyl, halogenoalkyl having 1 or 2 carbon atoms and 1 to 5 identical or different halogen atoms from the group consisting of fluorine, chlorine and bromine, or represents methoxymethyl, methoxyethyl, cyclopropyl or cyclohexyl,
R² and R³ either independently of one another in each case represent hydrogen, methyl, ethyl, n- or i-propyl, methoxycarbonyl or ethoxycarbonyl or together with the nitrogen atom to which they are bonded represent a straight-chain or branched alkylideneimino radical having 1 to 6 carbon atoms, or a benzylideneimino radical which is optionally mono- to trisubstituted by identical or different substituents, suitable substituents .in the aryl moiety being:
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, trifluoromethyl, difluoromethyl, trifluoromethoxy, difluoromethoxy, trifluoromethylthio, trifluoromethylsulphinyl, trifluoromethylsulphonyl,
R⁴ either represents hydrogen, cyano, methyl, ethyl, n- or i-propyl, n-, i-, s- or t-butyl, allyl, propargyl or represents straight-chain or branched halogenoalkyl having 1 to 6 carbon atoms and 1 to 13 identical or different halogen atoms from the group consisting of fluorine, chlorine and bromine and
R⁵ represents halogen, methyl, ethyl, n- or i-propyl, n-, i-, s- or t-butyl or straight-chain or branched halogenoalkyl having 1 to 6 carbon atoms and 1 to 13 identical or different halogen atoms from the group consisting of fluorine, chlorine and bromine or
R⁴ and R⁵ together with the carbon atom to which they are bonded represent cycloalkyl having 3 to 6 carbon atoms,
R⁶ represents hydrogen or straight-chain or branched alkyl having 1 to 7 carbon atoms which is optionally mono- to trisubstituted by identical or different substituents, suitable substituents being:
halogen from the group consisting of fluorine, chlorine and bromine, cyano, cycloalkyl having 3 to 6 carbon atoms, or phenyl, phenoxy, phenylthio or phenylamino, each of which is optionally monoto trisubstituted by identical or different substituents, heteroaryl, heteroaryloxy, heteroarylthio or heteroarylamino, each of which has 1 to 5 carbon atoms and 1 to 3 identical or different hetero atoms from the group consisting of nitrogen, oxygen and sulphur in the heteroaryl moiety and each of which is mono- to trisubstituted by identical or different substituents, suitable phenyl or heteroaryl substituents in each case being: fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, trifluoromethyl, difluoromethyl, trifluoromethoxy, difluoromethoxy, trifluoromethylthio, trifluoromethylsulphinyl, trifluoromethylsulphonyl,
R⁶ furthermore represents phenyl or heteroaryl having 1 to 5 carbon atoms and 1 to 3 identical or different hetero atoms from the group consisting of nitrogen, oxygen and sulphur, each of these phenyl or heteroaryl optionally being mono- to trisubstituted by identical or different substituents, suitable phenyl and heteroaryl substituents, respectively, in each case being: fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, methylsulphinyl, methylsulphonyl, trifluoromethyl, difluoromethyl, trifluoromethyl, difluoromethoxy, trifluoromethylthio trifluoromethylsulphinyl, trifluoromethylsulphonyl,
X represents oxygen or sulphur and
Z represents oxygen or sulphur.

2. Process for the preparation of substituted triazolinones according to Claim 1, characterized in that
a) activated (thio)carbonyltriazolinones of the formula (II) 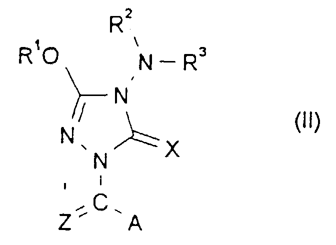 in which
R¹, R², R³, X and Z have the meanings given in Claim 1 and
A represents an activating group,
are reacted with amines of the formula (III) 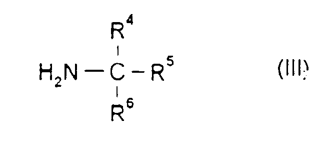 in which
R⁴, R⁵ and R⁶ have the meanings given in Claim 1,
if appropriate in the presence of a diluent and if appropriate in the presence of a reaction auxiliary, or in that
b) triazolinones which are unsubstituted in the 1-position and have the formula (IV) 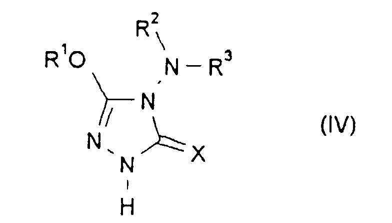 in which
R¹, R², R³ and X have the meanings given in Claim 1,
are reacted with iso(thio)cyanates of the formula (V) 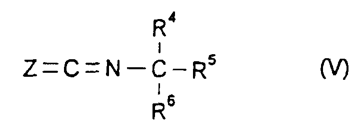 in which
R⁴, R⁵, R⁶ and Z have the meanings given in Claim 1,
if appropriate in the presence of a diluent and if appropriate in the presence of a reaction auxiliary, or in that
c) substituted triazolinones of the formula (Ia) 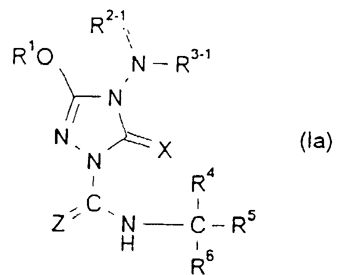 in which
R¹, R⁴, R⁵, R⁶, X and Z have the meanings given in Claim 1 and
R²⁻¹ and R³⁻¹ together with the nitrogen atom to which they are bonded represent an optionally substituted alkylideneimino radical having 1 to 6 carbon atoms,
are reacted with acid, if appropriate in the presence of a diluent and if appropriate in the presence of a reaction auxiliary, or in that
d) substituted triazolinones of the formula (Ib) 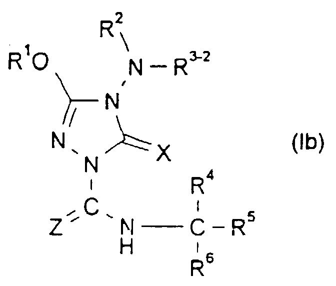 in which
R¹, R², R⁴, R⁵, R⁶, X and Z have the meanings given in Claim 1 and
R³⁻² represents methoxycarbonyl or ethoxycarbonyl
are hydrolysed, if appropriate in the presence of a diluent and if appropriate in the presence of a reaction auxiliary, and subsequently subjected to thermal decarboxylation.

3. Herbicidal agent, characterized in that it contains at least one substituted triazolinone according to Claim 1.

4. Method of combating undesired plants, characterized in that substituted triazolinones according to Claim 1 are allowed to act on plants and/or their environment.

5. Use of substituted triazolinones according to Claim 1 for combating undesired plants.

6. Process for the preparation of herbicidal agents, characterized in that substituted triazolinones according to Claim 1 are mixed with extenders and/or surface-active substances.

7. Substituted triazolinones of the formula (Ia) 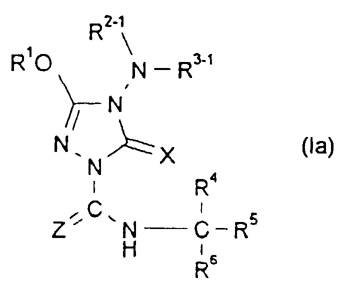 in which
R¹, R⁴, R⁵, R⁶, X and Z have the meanings given in Claim 1 and
R²⁻¹ and R³⁻¹ together with the nitrogen atom to which they are bonded represent a straight-chain or branched alkylideneimino radical having 1 to 6 carbon atoms.

8. Substituted triazolinones of the formula (Ib) 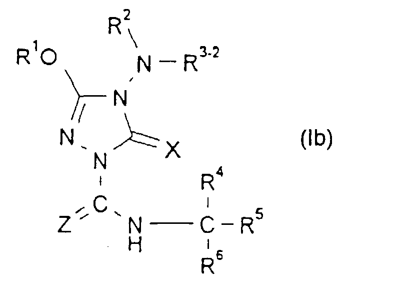 in which
R¹, R², R⁴, R⁵, R⁶, X and Z have the meanings given in Claim 1 and
R³⁻² represents methoxycarbonyl or ethoxycarbonyl.

## Revendications

1. Triazolinones substituées de formule générale (I) 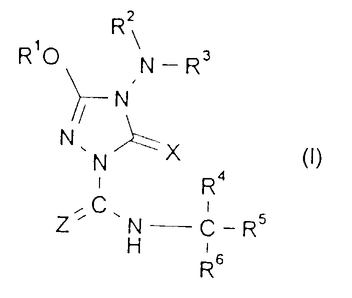 dans laquelle
R¹ est un groupe méthyle, éthyle, n-propyle, isopropyle, n-butyle, isobutyle, sec.-butyle ou tertio-butyle, un groupe halogénalkyle ayant 1 ou 2 atomes de carbone et 1 à 5 atomes d'halogènes identiques ou différents choisis dans le groupe fluor, chlore et brome, un groupe méthoxyméthyle, méthoxyéthyle, un groupe cyclopropyle ou cyclohexyle,
R² et R³ représentent chacun, indépendamment l'un de l'autre, l'hydrogène, un groupe méthyle, éthyle, n-propyle, isopropyle, méthoxycarbonyle ou éthoxycarbonyle ou forment conjointement avec l'atome d'azote auquel ils sont liés un reste alkylidène-imino linéaire ou ramifié ayant 1 à 6 atomes de carbone ou un reste benzylidène-imino éventuellement substitué 1 ou 3 fois identiques ou différentes, les substituants considérés dans la partie aryle étant :
fluor, chlore, brome, cyano, nitro, méthyle, éthyle, n-propyle, isopropyle, n-butyle, isobutyle, sec.-butyle, tertio-butyle, méthoxy, éthoxy, n-propoxy, iso-propoxy, n-butoxy, isobutoxy, sec.-butoxy, tertio-butoxy, méthylthio, éthylthio, méthylsulfinyle, méthylsulfonyle, trifluorométhyle, difluorométhyle, trifluorométhoxy, difluorométhoxy, trifluorométhylthio, trifluorométhyl-sulfinyle, trifluorométhylsulfonyle,
R⁴ représente l'hydrogène ou un groupe cyano, méthyle, éthyle, n-propyle, isopropyle, n-butyle, isobutyle, sec.-butyle, tertio-butyle, allyle, propargyle ou un groupe halogénalkyle linéaire ou ramifié ayant 1 à 6 atomes de carbone et 1 à 13 atomes d'halogènes identiques ou différents choisis dans le groupe fluor, chlore, et brome et
R⁵ est l'hydrogène, un groupe méthyle, éthyle, n-propyle, isopropyle, n-butyle, isobutyle, sec.-butyle, tertio-butyle ou un groupe halogénalkyle linéaire ou ramifié ayant 1 à 6 atomes de carbone et 1 à 13 atomes d'halogènes identiques ou différents choisis dans le groupe fluor, chlore et brome, ou bien
R⁴ et R⁵ forment, conjointement avec l'atome de carbone auquel ils sont liés, un groupe cycloalkyle de 3 à 6 atomes de carbone,
R⁶ est l'hydrogène ou un groupe alkyle linéaire ou ramifié de 1 à 7 atomes de carbone, portant le cas échéant 1 à 3 substituants identiques ou différents, les substituants considérés étant : halogène du groupe fluor, chlore et brome, cyano, cycloalkyle ayant 3 à 6 atomes de carbone ou phényle, phénoxy, phénylthio, phénylamino portant chacun le cas échéant 1 à 3 substituants identiques ou différents, hétéroaryle, hétéroaryloxy, hétéroarylthio ou hétéroarylamino portant chacun 1 à 3 substituants identiques ou différents et ayant chacun 1 à 5 atomes de carbone et 1 à 3 hétéroatomes identiques ou différents choisis dans le groupe azote, oxygène et soufre dans la partie hétéroaryle, les substituants considérés du groupe phényle et du groupe hétéroaryle étant : fluor, chlore, brome, cyano, nitro, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, trifluorométhyle, difluorométhyle, trifluorométhoxy, difluorométhoxy, trifluorométhylthio, trifluorométhylsulfinyle, trifluorométhylsulfonyle,
R⁶ représente en outre un groupe phényle ou un groupe hétéroaryle portant chacun le cas échéant 1 à 3 substituants identiques ou différents et ayant 1 à 5 atomes de carbone et 1 à 3 hétéroatomes identiques ou différents choisis dans le groupe azote, oxygène et soufre, les substituants considérés du groupe phényle et du groupe hétéroaryle étant les suivants : fluor, chlore, brome, cyano, nitro, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, méthylsulfinyle, méthylsulfonyle, trifluorométhyle, difluorométhyle, trifluorométhoxy, difluorométhoxy, trifluoro-méthylthio, trifluorométhylsulfinyle, trifluoro-méthylsulfonyle,
X représente de l'oxygène ou du soufre et
Z représente de l'oxygène ou du soufre.

2. Procédé de production de triazolinones substituées suivant la revendication 1, caractérisé en ce que
a) on fait réagir des (thio)carbonyltriazolinones activées de formule (II) 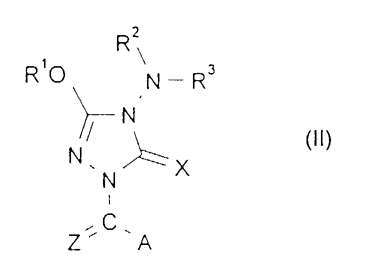 dans laquelle
R¹, R², R³, X et Z ont les définitions indiquées dans la revendication 1 et
A est un groupe activateur,
avec des amines de formule (III) 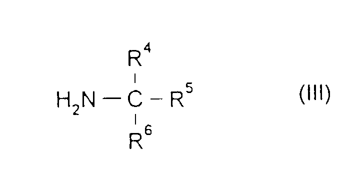 dans laquelle
R⁴, R⁵ et R⁶ ont les définitions indiquées dans la revendication 1,
le cas échéant en présence d'un diluant et en la présence éventuelle d'une substance auxiliaire de réaction, ou bien
b) on fait réagir des triazolinones non substituées en position 1 de formule (IV) 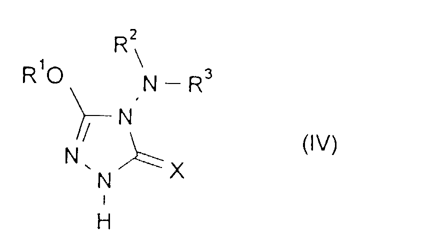 dans laquelle
R¹, R², R³ et X ont les définitions indiquées dans la revendication 1,
avec des iso(thio)cyanates de formule (V), 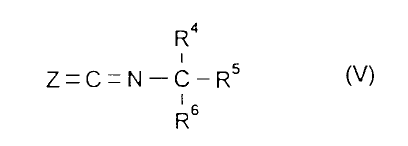 dans laquelle
R⁴, R⁵, R⁶ et Z ont les définitions indiquées dans la revendication 1,
le cas échéant en présence d'un diluant et en la présence éventuelle d'une substance auxiliaire de réaction, ou bien
c) on fait réagir des triazolinones substituées de formule (Ia) 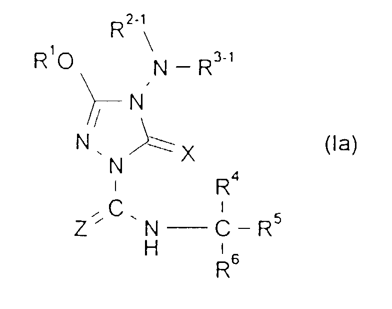 dans laquelle
R¹, R⁴, R⁵, R⁶, X et Z ont les définitions indiquées dans la revendication 1 et
R²⁻¹ et R³⁻¹ forment conjointement avec l'atome d'azote auquel ils sont liés un reste alkylidène-imino éventuellement substitué ayant 1 à 6 atomes de carbone,
avec un acide, le cas échéant en présence d'un diluant et en la présence éventuelle d'une substance auxiliaire de réaction, ou bien
d) on saponifie des triazolinones substituées de formule (Ib) 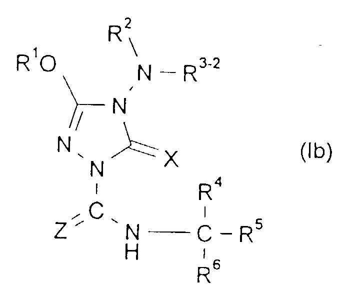 dans laquelle
R¹, R², R⁴, R⁵, R⁶, X et Z ont les défini tions indiquées dans la revendication 1 et
R³⁻² représente un groupe alkoxycarbonyle, méthoxycarbonyle ou éthoxycarbonyle,
le cas échéant en présence d'un diluant et en la présence éventuelle d'une substance auxiliaire de réaction puis on effectue une décarboxylation thermique.

3. Compositions herbicides, caractérisées par une teneur en au moins une triazolinone substituée suivant la revendication 1.

4. Procédé pour combattre des plantes indésirables, caractérisé en ce qu'on fait agir des triazolinones substituées suivant la revendication 1 sur des plantes et/ou sur leur milieu.

5. Utilisation de triazolinones substituées suivant la revendication 1 pour combattre des plantes indésirables.

6. Procédé de préparation de compositions herbicides, caractérisé en ce qu'on mélange des triazolinones substituées suivant la revendication 1 avec des diluants et/où des substances tensio-actives.

7. Triazolinones substituées de formule (Ia) 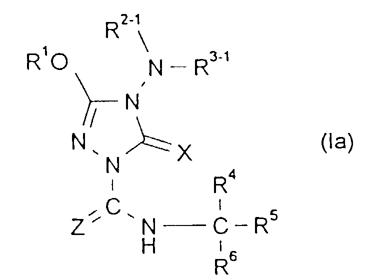 dans laquelle
R¹, R⁴, R⁵, R⁶, X et Z ont les définitions indiquées dans la revendication 1, et
R²⁻¹ et R³⁻¹ forment, conjointement avec l'atome d'azote auquel ils sont liés, un reste alkylidène-imino linéaire ou ramifié ayant 1 à 6 atomes de carbone.

8. Triazolinones substituées de formule (Ib) 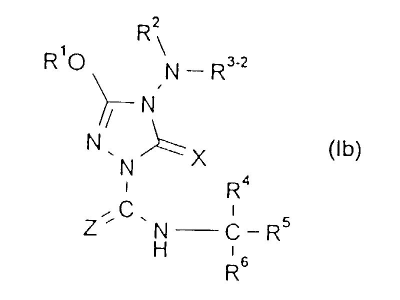 dans laquelle
R¹, R², R⁴, R⁵, R⁶, X et Z ont les définitions indiquées dans la revendication 1, et
R³⁻² représente un groupe méthoxycarbonyle ou éthoxycarbonyle.
